# EUROPÄISCHE PATENTSCHRIFT

(11) **EP 0 345 713 B1**
(45) Veröffentlichungstag und Bekanntmachung des Hinweises auf die Patenterteilung: **28.09.1994**
(21) Anmeldenummer: 89110167.7
(22) Anmeldetag: 05.06.1989
(51) Int. Cl.: A61B 1/12, A61L 2/26, A61M 16/00

(54) **Reinigungs- und Desinfektionsmaschine für medizinische Geräte und Instrumente, insbesondere für Anästhesieschläuche, Katheter und Endoskope**
Cleaning and disinfection apparatus for medical devices, particularly for anaesthesia tubes, catheters and endoscopes
Appareil de nettoyage et de désinfection pour instruments médicaux, en particulier tubulures d'anesthésie, cathéters et endoscopes

(30) Priorität: 06.06.1988 DE 3819257
(43) Veröffentlichungstag der Anmeldung: 13.12.1989
(73) Patentinhaber: Biermaier, Hans, 86316 Derching (DE)
(72) Erfinder: Biermaier, Hans, 86316 Derching (DE)
(74) Vertreter: von Bülow, Tam, Dr.

(56) Entgegenhaltungen:
- EP-A- 0 089 605
- DE-A- 3 710 517
- DE-U- 8 429 231

## Beschreibung

Die Erfindung betrifft eine Reinigungs- und Desinfektionsvorrichtung für medizinische Gerate und Instrumente, insbesondere für Anästhesieschläuche, Katheter und Endoskope gemäß dem Oberbegriff des Patentanspruches 1. Eine derartige Vorrichtung, die auch als Desinfektions-Spülvorrichtung bezeichnet wird, ist aus der EP-A-0 089 605 bekannt. In der älteren, nicht vorveröffentlichten DE-A1- 37 10 517 wird ein Endoskop im Inneren der Spülvorrichtung teilweise in ein Rohr gesteckt, durch welches Reinigungsflüssigkeit unter Druck geleitet wird, wodurch die Reinigungsbedingungen verbessert werden. Der Kopf des Endoskops, der nicht mit Reinigungsflüssigkeit in Berührung kommen soll, wird dabei in einem Formteil gehalten, das gegenüber dem Waschraum weitestgehend flüssigkeitsdicht ist. Zusätzlich wird dieses Formteil unter Druckluft gesetzt, wodurch ein Eindringen von Reinigungsflüssigkeit verhindert wird.

Mit dieser bekannten Vorrichtung werden zwar Endoskope sehr gut gereinigt. Problematisch ist jedoch weiterhin der Transport von gereinigten und desinfizierten Endoskopen innerhalb der Patientenräume. Werden Endoskope aus der Desinfektions-Spülvorrichtung entnommen, so sind sie ab diesem Moment nicht mehr gegen erneute Kontamination geschützt. Noch schlimmer ist der Transport der gebrauchten, also kontaminerten Geräte zur Desinfektions-Spülvorrichtung, da auf diesem Wege die gesamte Umgebung einschließlich Patienten und Personal einer Gefahr der Kontamination ausgesetzt ist.

Aufgabe der Erfindung ist es daher, die Vorrichtung der eingangs genannten Art dahingehend zu verbessern, daß die zu reinigenden Gegenstände auf dem Weg von und/oder zur Vorrichtung weder selbst kontaminiert werden können noch andere Gegenstände kontaminieren.

Diese Aufgabe wird bei der gattungsbildenden Vorrichtung durch die Merkmale des kennzeichnenden Teiles des Patentanspruches 1 gelöst. Vorteilhafte Ausgestaltungen und Weiterbildungen der Erfindung sind den Unteransprüchen zu entnehmen.

Der Grundgedanke der Erfindung besteht darin, die zu reinigenden bzw. gereinigten Gegenstände in einem geschlossenen Behälter zu transportieren, der während des Reinigungs- und Desinfektionsvorganges an die Vorrichtung angeschlossen wird. Das medizinische Gerät bleibt also mit Ausnahme der kurzen Zeitdauer seines Einsatzes am Patienten stets in dem Behälter, vor allem auch während des Reinigungs- und Desinfektionsvorganges. Nach der Reinigung wird der komplette Behälter aus der Vorrichtung entnommen und bis zum Patienten transportiert. Auf diesem Wege sind also die medizinischen Geräte absolut sicher gegen Kontamination geschützt und können so auch über längere Zeit gelagert werden. Umgekehrt wird das durch den Einsatz verschmutzte Gerät unmittelbar nach Gebrauch wieder in den Behälter eingesetzt. Der Behälter wird verschlossen und dann zurück zur Desinfektions-Spülvorrichtung transportiert. Auch auf diesem Rückweg kann also das selbst-kontaminierte Gerät nicht seine Umgebung kontaminieren.

Die Behälter selbst werden über Kupplungen, vorzugsweise Schnellkupplungen an das Waschsystem, das die Zufuhr und Abfuhr von Reinigungs- und Desinfektionsflüssigkeit beinhaltet, angeschlossen; ggf. auch an ein Druckluft- und/oder Vakuumsystem, das während bestimmter Phasen des Reinigungsvorganges benötigt wird.

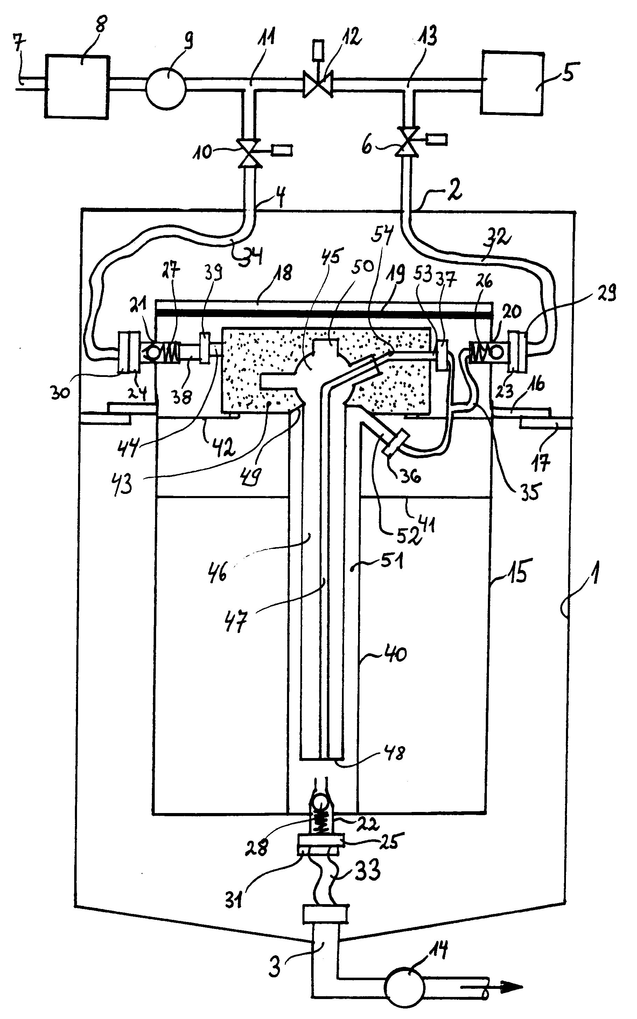

Im folgenden wird die Erfindung anhand eines Ausführungsbeispieles im Zusammenhang mit der einzigen Zeichnung erläutert, die schematisch die wichtigsten Teile der Reinigungs- und Desinfektionsvorrichtung nach der Erfindung zeigt.

Ein Waschraum 1 einer Reinigungs- und Desinfektionsvorrichtung besitzt mindestens eine Zufuhrleitung 2 für Reinigungs- und Desinfektionsflüssigkeiten, mindestens eine Abfuhrleitung 3, über welche alle Flüssigkeiten aus dem Waschraum abgeführt werden, sowie eine Druckluftzufuhrleitung 4, über die steril gefilterte Druckluft in den Waschraum 1 geleitet wird.

Ein Versorgungssystem für Reinigungs- und Desinfektionsflüssigkeiten (also z. B. mit Spülmittel versetztes Wasser, Desinfektionsflüssigkeit etc.) ist mit dem Bezugszeichen 5 angedeutet. Von dort gelangen die genannten Flüssigkeiteen über ein Magnetventil 6 zur Zufuhrleitung 2. Das Druckluftsystem beginnt mit einem Lufteinlaß 7, von wo die Luft über ein Sterilfilter 8 mittels einer Pumpe 9 über ein Magnetventil 10 zu der Druckluftzufuhrleitung 4 gelangt. Zwischen der Pumpe 9 und dem Magnetventil 10 ist ein T-Stück 11, das Druckluft auch über ein Magnetventil 12 zu dem Flüssigkeits-System leiten kann und zwar zu einem T-Stück 13, über welches dann wiederum die Verbindung zu dem Magnetventil 6 und zur Zufuhrleitung 2 geschaffen wird. Auf diesem Wege kann Druckluft auch zu dem Flüssigkeits-System geleitet werden, was besonders wichtig ist, wenn man nach dem Reinigungsvorgang Wasserreste mit Druckluft ausblasen will. Gerade bei Endoskopen mit teilweise kapillarartig dünnen Hohlräumen lassen sich Flüssigkeitsreste nur durch Druckluft entfernen. Auch kann das Druckluftsystem durch Aufheizen der Druckluft zum Trocknen und Sterilisieren eingesetzt werden.

An der Abfuhrleitung 3 ist eine Pumpe 14 angeschlossen, die unter Umständen während des gesamten Waschvorganges läuft und ein Vakuum erzeugt, so daß ständig ein Druckgefälle von oben nach unten in der Vorrichtung herrscht. Hierdurch werden die benötigten Flüssigkeiten schneller durch die Vorrichtung geleitet, was die Reinigungswirkung verbessert.

Im Inneren des Waschraumes 1 ist ein Behälter 15 zu sehen, der mittels seitlicher Tragarme 16 auf Schienen 17 gehalten ist und der das zu reinigende Gut enthält. Der Behälter 15 besitzt einen öffenbaren Deckel 18, mit welchem der Behälter dicht verschlossen werden kann. Die Abdichtung kan beispielsweise durch eine Gummidichtung 19 erfolgen. Der Deckel kann als Klapp- oder Schraubdeckel ausgebildet sein. Die Öffnung muß so groß sein, daß das Waschgut leicht eingesetzt werden kann. Der Behälter 15 besitzt im dargestellten Ausführungsbeispiel 3 weitere Öffnungen, nämlich einen Einlaß 20 für Reinigungsflüssigkeit, einen Einlaß 21 für Druckluft und einen Auslaß 22. Diese drei Öffnungen 20, 21 und 22 können als Rohrstutzen ausgebildet sein, an deren äußeren Enden jeweils eine Kupplung 23, 24 bzw. 25 angebracht ist, über die Leitungen angekuppelt werden können. Es kann sich um einfache Steckkupplungen handeln, auf die ein flexibler Schlauch aufgeschoben werden kann. Es kann sich auch um Schnellkupplungen, wie z. B. Bajonettkupplungen handeln. Es können auch automatisch arbeitende Kupplungen vorgesehen sein, die so am Behälter angebracht sind, daß sie beim Einschieben des Behälters automatisch an Anschlüsse des Waschraumes ankoppeln. Die drei zusätzlichen Behälteröffnungen 20, 21 und 22 sind jeweils mit Rückschlagklappen bzw. -ventilen 26, 27 bzw. 28 ausgestattet, wobei die Rückschlagventile 26 und 27 in Einlaßrichtung öffnen, während das Ventil 28 in Auslaßrichtung öffnet. Solange diese Ventile nicht in ihrer Durchlaßrichtung mit Druck beaufschlagt sind, schließen sie automatisch und unterbinden damit jeglichen Durchtritt von Bakterien usw. Dadurch sind insbesondere während des Transportes alle Ventile geschlossen, so daß bei geschlossenem Deckel 18 der Behälter absolut dicht ist.

An die drei Kupplungen 23, 24 bzw. 25 werden nun über Kupplungsgegenstücke 29, 30 bzw. 31 Leitungen 32, 34 bzw. 33 angekuppelt. Die Leitung 32 führt die Reinigungsflüssigkeit zu dem Behälter. Die Leitung 33 führt die verbrauchte Reinigungsflüssigkeit ab und die Leitung 34 führt die Druckluft zu. Im dargestellten Ausführungsbeispiel sind die Leitungen 32, 33 und 34 als flexible Schläuche ausgebildet, die innerhalb des Waschraumes zu unterschiedlichen Stellen gelangen, so daß auch Behälter bzw. Kassetten unterschiedlicher Größe verwendet werden können.

Das innere des Behälters ist dem jeweils zu reinigenden Gut angepaßt. So können verschiedene Austrittsdüsen für Reinigungsflüssigkeit vorgesehen sein. Es könneninsbesondere für die Reinigung von Schläuchen und Endoskopen - auch spezielle Anschlüsse vorgesehen sein. Im dargestellten Ausführungsbeispiel ist der Behälter 15 für die Reinigung eines Endoskopes ausgebildet. Hierzu ist der Auslaß des Rückschlagventiles 26 an einen Schlauch 35 angeschlossen, der sich verzweigt und in zwei Kupplungen 36 und 37 mündet, womit die Reinigungsflüssigkeit auf zwei Wege verteilt wird, wie es zur Reinigung von Arbeitskanal und Außenseite eines Endoskopes erforderlich ist. Der Auslaß des Ventiles 27 ist mit einem Schlauch 38 verbunden, der ebenfalls in eine Kupplung 39 mündet. Schließlich ist der Auslaß des Ventiles 28 ebenfalls mit einer Kupplung 25 verbunden, an die über eine Kupplung 31 der Schlauch 33 angeschlossen werden kann. In spezieller Anpassung an die Reinigung von Schläuchen und Endoskopen ist im Inneren des Behälters 15 ein Rohr 40 vorgesehen, das den Schlauch oder das Endoskop aufnimmt. Dieses Rohr ist über eine Halterung 41 an der Behälterinnenwand abgestützt. Dieses Rohr mündet in spezieller Anpassung an Endoskope mit "nicht waschbarem Kopf" an seiner Oberseite in einen Raum aus Dichtungsmaterial 43, das in sich porös ist und einen Hohlraum aufweist, der an die Form des Endoskopkopfes 45 angepaßt ist. Dieses poröse Dichtungsmaterial 43 besitzt einen Anschlußstutzen 44 für Druckluft, an den also die Kupplung 39 angeschlossen werden kann. Damit kann das Dichtungsmaterial unter Druck gesetzt werden, so daß Reinigungsflüssigkeit nicht zu dem Endoskopkopf 45 gelangen kann.

Ein flexibles Endoskoprohr 46, das in seinem Inneren einen Arbeitskanal 47 aufweist, ist nun in das Rohr 40 eingeschoben, wobei zwischen dem Endoskoprohr 46 und der Innenwand des Rohres 40 ein Zwischenraum 51 gebildet ist. Das distale Ende 48 des Endoskoprohres 46 ist im übrigen kürzer als das Rohr 40. Der Zwischenraum 51 mündet an seinem oberen Ende in einen Anschlußstutzen 52, an welchen die Kupplung 36 angeschlossen werden kann. Der Arbeitskanal 47 mündet über eine anzuschließende Leitung 54 in den Anschlußstutzen 53, an den die Kupplung 37 angeschlossen werden kann. Auf diese Weise können der Arbeitskanal 47 und der Zwischenraum 51 mit der Reinigungs- und der Desinfektionsflüssigkeit beaufschlagt werden. Diese unter Druck stehende Flüssigkeit fließt durch diese Räume und wird durch den Unterdruck am Auslaßventil 28 abtransportiert. Dadurch, daß das Rohr 40 länger ist als das Endoskoprohr 46, biidet sich am distalen Ende 48 des Endoskoprohres 46 eine Abrißkante, wodurch der Unterdruck im Arbeitskanal 47 und im Zwischenraum 51 noch vergrößert wird. Durch die Beaufschlagung des porösen Dichtungsmaterials 43 mit Druckluft bildet sich am Einlaß des Zwischenraumes 41 ein Druckraum 49, wodurch die Reinigungsflüssigkeit zusätzlich zu ihrem eigenen Druck noch mit dem Druck der Druckluft beaufschlagt wird.

Für die Reinigung sehr langer Schläuche oder Endoskope kann das Rohr 40 natürlich auch die Form einer Spirale haben, da der Waschraum der üblichen Vorrichtungn nur eine begrenzte Höhe hat.

Im übrigen ist das Innere des Behälters 15 natürlich den jeweiligen Gegebenheiten anzupassen. Für die Reinigung von Reagenzgläsern, Flaschen etc. können mehrere dornartige Halterungen mit Düsen vorgesehen sein. Auch können im Inneren des Behälters Rohrsysteme mit Reinigungsdüsen verlegt sein, wie es der jeweilige Anwendungsfall fordert. Selbstverständlich ist es auch möglich, mehrere Behälter 15 gleichzeitig in die Vorrichtung einzusetzen. In diesem Falle sind entweder mehrere Schläuche für den separaten Anschluß jedes einzelnen Behälters vorzusehen oder die Behälter können jeweils zusätzlich Rohrverbindungen haben, so daß ein Behälter an den anderen Behälter angekuppelt werden kann, womit dann die benötigten Anschlüsse hergestellt werden. Die Form des Behälters ist natürlich auch frei wählbar. Im gezeigten Ausführungsbeispiel könnte der Behälter auch unmittelbar durch das Rohr 40 und das Dichtungsmaterial 43 für den Kopf des Endoskopes bestehen. wobei das Dichtungsmaterial dann an seiner Außenseite eine dichte Beschichtung, beispielsweise aus Kunststoff hätte, wobei das so gebildete Gehäuse seitlich aufklappbar zu gestalten wäre. Wichtig ist nur, daß der so gebildete Behälter absolut dicht gegen Kontamination ist.

Nach einer weiteren, in der Zeichnung nicht dargestellten Modifikation der Erfindung bildet der Behälter 15 unmittelbar den Waschraum der Vorrichtung. Die Vorrichtung hat dann zwei Module, nämlich den auswechselbaren Waschraum (Behälter) und das komplette Waschsystem mit Steuereinheiten, Pumpen, Heizungen, Überwachungen und Trockengebläse sowie die Einheit der mittels Schnellverschlüssen anzukoppelnden "Waschkammer", die durch den bzw. die Behälter gebildet wird. Hierdurch erhält man auch eine Verbesserung der Wartungsmöglichkeiten, da die nicht verschleißenden und damit nicht reparaturanfälligen Teile, wie die Waschkammer bzw. der Behälter, komplett von den Verschleißteilen getrennt sind. Damit kann man eine Folgekosten günstigere Vorrichtung herstellen, da der komplette störanfällige Teil vom Endbenutzer selbst ausgewechselt werden kann.

Weiterhin ist hervorzuheben, daß alle Anschlüsse (Systemreinigung, Systemdruckluft und Systementsorgung) so angeordnet sind, daß alle Medien (Reinigungsflüssigkeit, Desinfektionsflüssigkeit, Druckluft) immer von oben nach unten durch die Vorrichtung strömen. Hierdurch wird einmal die Strömungsgeschwindigkeit weiter erhöht. Zum anderen bleiben weniger Flüssigkeitsreste in der Vorrichtung. Falls solche doch noch vorhanden sind, werden diese dadurch ausgeblasen, daß Druckluft auf das System Reinigung geschaltet wird.

Durch die ständige Bereitstellung eines Vakuums am Auslaßende der Vorrichtung erhält man zusätzlich die günstige Möglichkeit, ohne Einsatz von Dosierpumpen die Menge der Reinigungs- und Desinfektionsmittelzusätze dosieren zu können. Durch diesen Unterdruck können im Zusammenwirken mit der zeitlichen Ansteuerung von Magnetventilen die Waschmittelzusätze nämlich unmittelbar aus einem Behälter gesaugt werden. Dadurch entfallen die sehr störanfälligen und meistens doch relativ ungenau arbeitenden Dosierpumpen.

Schließlich ist darauf hinzuweisen, daß - obwohl in der Figur nicht dargestellt - auch Einrichtungen vorgesehen sein können, um die Behälteraußenseite zu waschen. Dies kann durch zusätzliche in den Waschraum 1 mündende Düsen erfolgen. Auch können herkömmliche Wascharme vorgesehen sein. In bestimmten Fällen können auch im Inneren des Behälters rotierende Wascharme herkömmlicher Bauart verwendet werden.

## Patentansprüche

1. Reinigungs- und Desinfektionsvorrichtung für medizinische Geräte und Instrumente (Gegenstände), insbesondere für Anästhesieschläuche, Katheter und Endoskope, mit mindestens einer kuppelbaren Zufuhrleitung für Reinigungsflüssigkeit und mindestens einer Abfuhrleitung für die Entsorgung verbrauchter Reinigungsflüssigkeit, gekennzeichnet durch folgende Merkmale:
a) Es ist ein in die Vorrichtung einsetzbarer und aus der Vorrichtung entnehmbarer Transport- und Reinigungsbehälter (15) zur Aufnahme der Gegenstände (46) vorgesehen.
b) Der Behälter (15) besitzt eine dicht verschließbare Öffnung (18) zum Beschicken und Entnehmen der zu reinigenden Gegenstände.
c) Der Behälter (15) hat mindestens einen Einlaß (20, 21) und einen Auslaß (22).
d) Der Einlaß (20) ist an die kuppelbare Zufuhrleitung (32) anschließbar.
e) Der Auslaß (22) ist an die Abfuhrleitung (33) kuppelbar.
f) Einlässe und Auslässe (20, 21, 22) sind durch Rückschlagklappen oder -ventile (26, 27, 28) verschließbar, wobei diese Klappen oder Ventile (26, 27, 28) in Ruhestellung geschlossen und durch ein unter Druck stehendes Fluid öffenbar sind.

2. Vorrichtung nach Anspruch 1, dadurch gekennzeichnet, daß der Behälter (15) aus Kunststoff oder Edelstahl besteht.

3. Vorrichtung nach Anspruch 1 oder 2, dadurch gekennzeichnet, daß die Zufuhr- und Abfuhrleitungen (32, 33, 34) durch beim Einschieben des Behälters (15) in die Vorrichtung automatisch arbeitende Schnellkupplungen (29, 30, 31) an den Behälter (15) anschließbar sind.

4. Vorrichtung nach einem der Ansprüche 1 bis 3, dadurch gekennzeichnet, daß mehrere Behälter (15) aneinander ankuppelbar sind, so daß alle Behälter mit den Zufuhr- und Abfuhrleitungen (32, 33, 34) in Strömungsverbindung stehen.

5. Vorrichtung nach einem der Ansprüche 1 bis 4, dadurch gekennzeichnet, daß die Behälter (15) an eine getrennte Druckluftleitung (34) anschließbar sind.

6. Vorrichtung nach einem der Ansprüche 1 bis 5, dadurch gekennzeichnet, daß die normalerweise die Reinigungsflüssigkeit führende Leitung (32) umschaltbar an das Reinigungssystem (5) oder an das Druckluftsystem (7, 8, 9) anschließbar ist.

7. Vorrichtung nach einem der Ansprüche 1 bis 6, dadurch gekennzeichnet, daß die Abfuhrleitungen (3, 33) an ein Unterdrucksystem (Pumpe 14) anschließbar ist.

8. Vorrichtung nach Anspruch 7, dadurch gekennzeichnet, daß die Dosierung der flüssigen Reinigungsmittel durch das Unterdrucksystem (Pumpe 14) in Zusammenwirken mit einem zeitlich angesteuerten Magnetventil (6) machbar ist.

9. Vorrichtung nach einem der Ansprüche 1 bis 8, dadurch gekennzeichnet, daß die Zufuhrleitungen (2,4) für Reinigungsflüssigkeit und Druckluft sowie für die Abfuhrleitung (3, 33) so angeordnet sind, daß alle Medien immer von oben nach unten durch den Behälter (15) strömbar sind.

10. Vorrichtung nach einem der Ansprüche 1 bis 9, dadurch gekennzeichnet, daß sie aus nur zwei trennbaren Modulen aufgebaut ist, wobei das erste Modul eine Steuereinheit, Pumpen, eine Heizung etc. enthält, während das zweite Module aus dem Behälter (15) besteht.

11. Vorrichtung nach Ansprüchen 1 bis 9, dadurch gekennzeichnet, daß sie mit einer Anschlußkupplung in Form einer Injektordüse versehen ist, die ein kontrolliertes Beimischen von Raumluft in die Waschflotte bewirkt, deren Wirkung die Reinigungskraft der Wasch- und Spüllauge verbessert.

## Claims

1. A cleaning and disinfecting device for medical apparatus and instruments (pieces of equipment), in particular for anaesthetic hoses, catheters and endoscopes, having at least one couplable supply line for cleaning fluid and having at least one discharge line for the removal as waste of spent cleaning fluid, characterised by the following features:
a) a transport and cleaning receptacle (15) for receiving the pieces of equipment (46) is provided which can be inserted into the device and removed therefrom.
b) the receptacle (15) has an imperviously closable opening (18) for inserting and removing the pieces of equipment to be cleaned.
c) the receptacle (15) has at least one inlet (20, 21) and an outlet (22).
d) the inlet (20) can be attached to the couplable supply line (32).
e) the outlet (22) can be coupled to the discharge line (33).
f) inlets and outlets (20, 21, 22) can be closed by non-return flaps or non-return valves (26, 27, 28), these flaps or valves (26, 27, 28) being closed in the rest position and being able to be opened by means of a fluid under pressure.

2. A device in accordance with Claim 1, characterised in that the receptacle (15) is composed of a plastics material or special steel.

3. A device in accordance with Claim 1 or 2, characterised in that the supply and discharge lines (32, 33, 34) can be attached to the receptacle (15) by means of quick-fitting couplings (29, 30, 31) operating automatically when the receptacle (15) is pushed into the device.

4. A device in accordance with any one of Claims 1 to 3, characterised in that a plurality of receptacles (15) can be coupled to one another so that all receptacles are in a flow connection with the supply and discharge lines (32, 33, 34).

5. A device in accordance with any one of Claims 1 to 4, characterised in that the receptacles (15) can be attached to a separate compressed-air line (34).

6. A device in accordance with any one of Claims 1 to 5, characterised in that the line (32) normally conveying the cleaning fluid can be attached, so as to be able to be changed over, to the cleaning system (5) or to the compressed-air system (7, 8, 9).

7. A device in accordance with any one of Claims 1 to 6, characterised in that the discharge lines (3, 33) can be attached to a vacuum system (pump 14).

8. A device in accordance with Claim 7, characterised in that the metering of the fluid cleaning-agent through the vacuum system (pump 14) can be effected in cooperation with a chronologically activated solenoid valve (6).

9. A device in accordance with any one of Claims 1 to 8, characterised in that the supply lines (2, 4) for cleaning fluid and compressed air as well as for the discharge line (3, 33) are arranged in such a manner that all media can always flow through the receptacle (15) from the top to the bottom.

10. A device in accordance with any one of Claims 1 to 9, characterised in that it is constructed of only two separable modules, the first module containing a control unit, pumps, a heating device etc., while the second module is composed of the receptacle (15).

11. A device in accordance with Claims 1 to 9, characterised in that it is provided with a connecting coupling in the form of an injector nozzle which effects a controlled mixing of ambient air with the washing liquid, the effect of this ambient air improving the cleaning power of the washing and rinsing liquor.

## Revendications

1. Appareil de nettoyage et de désinfection pour appareils et instruments médicaux (objets), notamment pour des tuyaux souples d'anesthésie, des cathéters et des endoscopes, comportant au moins un conduit d'admission raccordable pour un liquide de nettoyage et au moins un conduit de décharge pour l'évacuation d'un liquide de nettoyage usé, caractérisé par les particularités suivantes:
a) il est prévu un récipient de transport et nettoyage (15) servant à recevoir les objets (46), pouvant être mis en place dans l'appareil et pouvant être sorti de l'appareil;
b) le récipient (15) comporte une ouverture (18) pouvant être obturée de façon étanche, pour l'introduction et l'enlèvement des objets à nettoyer,
c) le récipient (15) comporte au moins une entrée (20, 21) et une sortie (22),
d) l'entrée (20) peut être reliée au conduit d'admission (32) raccordable,
e) la sortie (22) peut être reliée au conduit de décharge (33),
f) des entrées et des sorties (20,21,22) peuvent être obturées par des clapets ou des soupapes anti-retour (26, 27, 28), ces clapets ou soupapes (26, 27, 28) étant fermés dans la position de repos et pouvant être ouverts par un fluide sous pression.

2. Appareil selon la revendication 1, caractérisé en ce que le récipient (15) se compose de matières plastiques ou d'acier allié.

3. Appareil selon la revendication 1 ou 2, caractérisé en ce que les conduits d'admission et de décharge (32, 33,34) doivent être raccordés au récipient (15) par des raccords rapides (29, 30, 31) opérant automatiquement lors de l'engagement du récipient (15) dans l'appareil.

4. Appareil selon l'une des revendications 1 à 3, caractérisé en ce que plusieurs récipients (15) peuvent être raccordés l'un avec l'autre de telle sorte que tous les récipients soient en liaison d'écoulement avec les conduits d'admission et de décharge (32, 33, 34).

5. Appareil selon l'une des revendications 1 à 4, caractérisé en ce que les récipients (15) peuvent être raccordés à un conduit d'air comprimé séparé (34).

6. Appareil selon l'une des revendications 1 à 5, caractérisé en ce que le conduit (32) canalisant normalement le liquide de nettoyage, peut être raccordé, avec possibilité d'inversion, au système de nettoyage (5) ou au système d'air comprimé (7, 8, 9).

7. Appareil selon l'une des revendications 1 à 6, caractérisé en ce que les conduits de décharge (3, 33) peuvent être raccordés à un système opérant en dépression (pompe 14).

8. Appareil selon la revendication 7, caractérisé en ce que le dosage de l'agent liquide de nettoyage peut être effectué par le système opérant en dépression (pompe 14) en coopération avec une soupape électromagnétique (6) pouvant être commandée temporellement.

9. Appareil selon l'une des revendications 1 à 8, caractérisé en ce que les conduits d'admission (2, 4) pour le liquide de nettoyage et l'air comprimé ainsi que le conduit de décharge (3, 33) sont disposés de telle sorte que tous les agents puissent toujours s'écouler à travers le récipient (15) du haut vers le bas.

10. Appareil selon l'une des revendications 1 à 9, caractérisé en ce qu'il est constitué de seulement deux modules séparables, le premier module contenant l'unité de commande, des pompes, un dispositif de chauffage, etc. tandis que le second module se compose du récipient (15).

11. Appareil selon les revendications 1 à 9, caractérisé en ce qu'il est pourvu d'un raccord d'accouplement se présentant sous la forme d'une buse d'injection, qui assure un mélange contrôlé d'air ambiant avec le liquide de lavage et dont l'action améliore le pouvoir de nettoyage de la lessive de lavage et de rinçage.
